# EUROPEAN PATENT SPECIFICATION

(11) **EP 4 299 066 B1**
(45) Date of publication and mention of the grant of the patent: **11.11.2026**
(21) Application number: 23181838.6
(22) Date of filing: 27.06.2023
(51) Int. Cl.: A61K 35/745, A61K 35/747, A61P 31/04, A61P 1/00, A23L 33/00, A23L 33/12, A23L 33/135, A23L 33/21, C12N 1/20, C12N 1/205

(54) **NOVEL BIFIDOBACTERIUM BREVE STRAIN**
NEUER BIFIDOBACTERIUM BREVE-STAMM
NOUVELLE SOUCHE DE BIFIDOBACTERIUM BREVE

(30) Priority: 27.06.2022 EP 22181253; 27.06.2022 EP 22181363; 27.06.2022 EP 22181345
(43) Date of publication of application: 03.01.2024
(73) Proprietor: Hipp & Co, 6072 Sachseln (CH)
(72) Inventor: Schaubeck, Monika, 84089 Aiglsbach (DE); Mader, Isabelle, 81927 München (DE); Juan Miguel, Rodríguez Gómez, 28770 Madrid (ES)
(74) Representative: Schiweck Weinzierl Koch Patentanwälte Partnerschaft mbB

(56) References cited:
- WO-A1-2007/045502
- WO-A1-2011/039328
- INSTITUTO DE INVESTIGACIÓN SANITARIA DEL PRINCIPADO DE ASTURIAS: "Memoria Científica 2020", 1 November 2020 (2020-11-01), pages 1 - 288, XP055977862, Retrieved from the Internet <URL:https://www.ispasturias.es/wp-content/uploads/2022/08/Memoria-Cientifica-ISPA-2020.pdf> [retrieved on 20221104]

## Description

### FIELD OF THE INVENTION

The present invention relates to a novel *Bifidobacterium breve* strain. Also included in the present invention is a composition, such as an infant formula, comprising the novel *Bifidobacterium breve* strain. The present invention also relates to the use of the *Bifidobacterium breve* strain for the manufacture of said composition.

### BACKGROUND OF THE INVENTION

Microbial colonization of the neonatal gut during birth is an essential event, as the establishment of a healthy gut microbiota is pivotal in priming the infant's immune system and therefore modulate risk to infections or allergies in later life. Human milk is the best possible nutrition for infants, as it also ensures optimized microbiota maturation due to its probiotic and prebiotic properties. However, when breastfeeding is not possible, infant formula should support similar development of the intestinal microbiota.

With respect to human newborn infants, it is still uncertain where and how they acquire beneficial gut bacteria, such as *Bifidocbacterium* spp. There is likewise ongoing research about which particular strains of these gut bacteria, including *Bifidobacterium* spp., are beneficial, e.g. for the newborn infants. WO2007/045502 for example teaches a composition comprising *Bifidobacterium breve* for stimulating the intestinal flora of infants delivered by caesarean section, who lack this and other bacteria compared to vaginal delivery newborns. Further, WO2011/039328 teaches a composition comprising *Bifidobacterium breve* for preventing infant inflammation later in life, wherein the B. *breve* strain is NCC 2950 - deposited as B. *breve* CNCM 1-3865. There is thus an ongoing need in the art to identify and provide novel gut bacteria that might be used in the formulation of infant formulae.

The technical problem underlying the present application is thus to provide a new gut bacteria strain which is suitable for infant formulae. The technical problem is solved by providing the embodiments reflected in the claims, described in the description, and illustrated in the examples and figures that follow.

### SUMMARY OF THE INVENTION

The present invention is set out in the appended set of claims. Specifically, the present invention provides a specific *Bifidobacterium breve* strain (short: *B. breve*), which has been deposited with the DSMZ under the accession number DSM 32583 and is called *inter alia* herein *B. breve* DSM 32583 or DSM 32583 or the like. Said particular strain was isolated from human breast milk and comprises specific unique features. Said particular strain DSM 32583 can also be comprised in a composition as defined herein.

Accordingly, in a first aspect, the present invention relates to a B. *breve* strain which is deposited with the DSMZ under the accession number DSM 32583. In a second aspect, the present invention relates to compositions, such as infant formulae, comprising said B. *breve* strain as mentioned elsewhere herein. In a third aspect, the present invention relates to the use of the *B. breve* strain DSM 32583 for the manufacture of compositions. In a fourth aspect, the present invention relates to a method for the manufacture of an infant formula comprising the step of formulating the B. *breve* strain DSM 32583 in a nutritional composition.

### BRIEF DESCRIPTION OF THE FIGURES

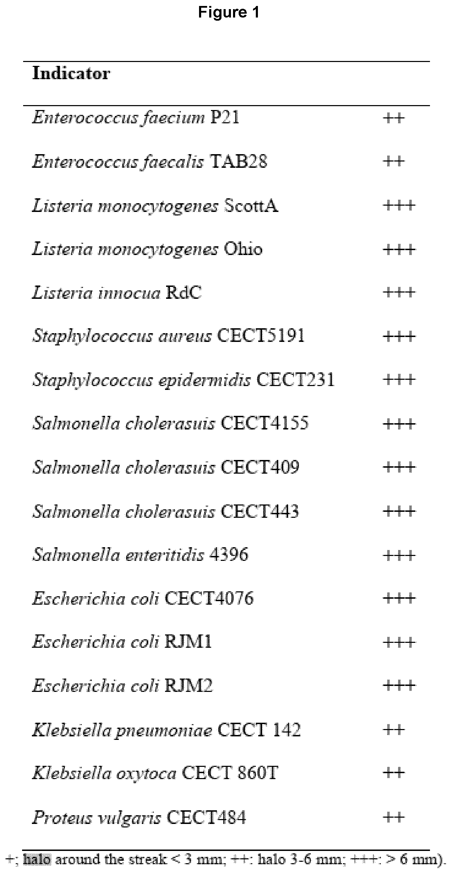

**Figure 1****:** Antimicrobial activity of the isolate *B*. *breve* DSM 32583.

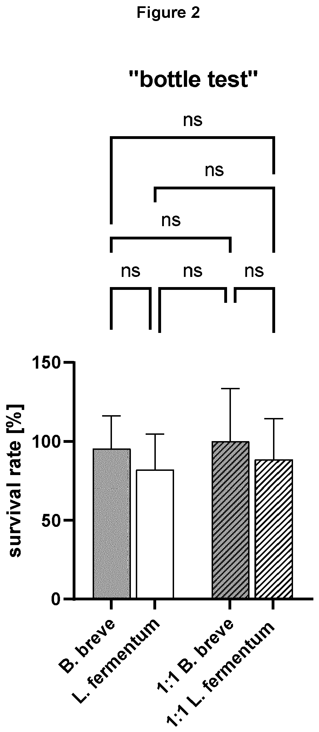

**Figure 2****:** Survival of *B*. *breve* DSM 32583, *L. fermentum* CECT 5716 or a combination of said two strains after infant's formula preparation ("bottle test": bacteria growth after shaking the formula in the bottle). The survival rate of B. *breve* DSM 32583 is about 93% or more. Non significant (short: ns) comparisons (p>0.05) are depicted by brackets.

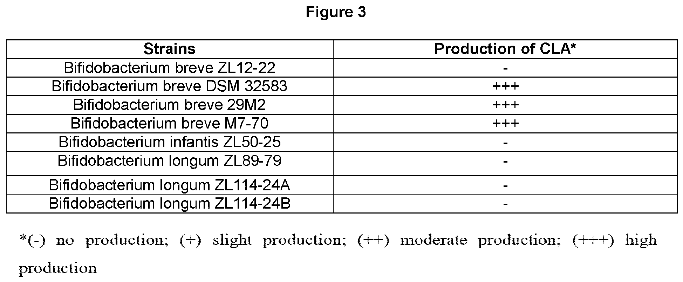

**Figure 3****:** CLA production level by the bifidobacterial strains screened.

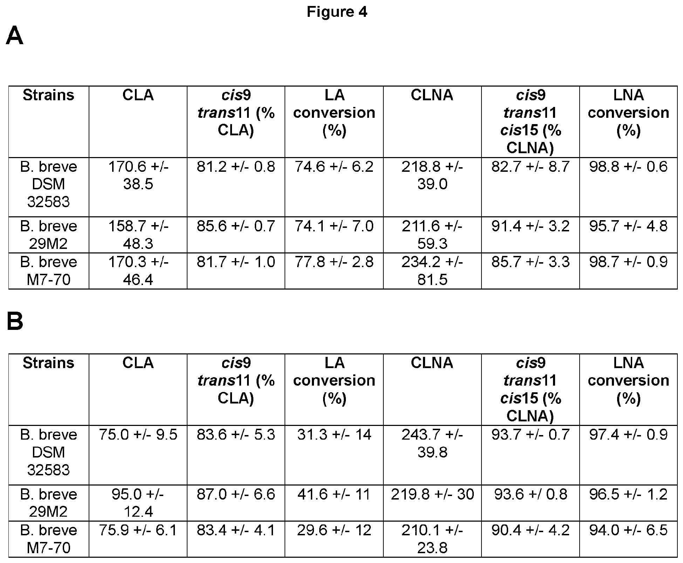

Figure 4**: (A)** CLA and CLNA production (µg/ml) and conversion (%) of LA and LNA by the bifidobacterial strains when growing in MRS-Cys. Values are means of triplicate experiments and standard deviation (± SD). **(B)** CLA and CLNA production (µg/ml) and conversion (%) of LA and LNA by *B. breve* strains when growing in skim milk. Values are means of triplicate experiments and standard deviation (± SD).

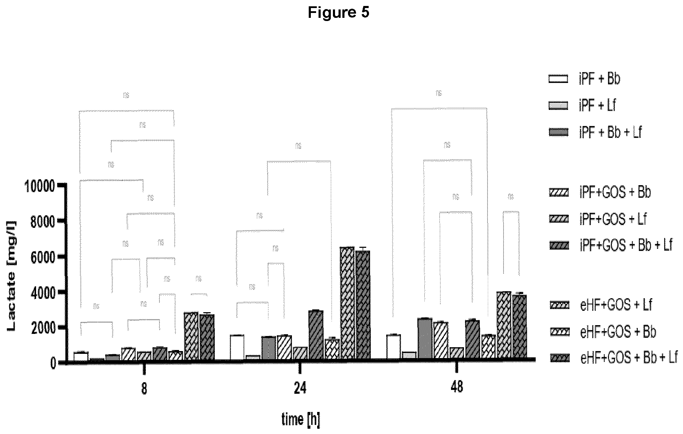

**Figure 5****:** Total lactate production after 8, 24 and 48 h by formula and by (*B. breve* DSM 32583 (short: Bb), *L. fermentum* (short: Lf), or combination (short: Bb + Lf)). Non significant (short: ns) comparisons (p>0.05) are depicted by brackets. No brackets means significant differences between the bars (p<0.05). iPF = intact protein formula; eHF = extensively hydrolysed formula.

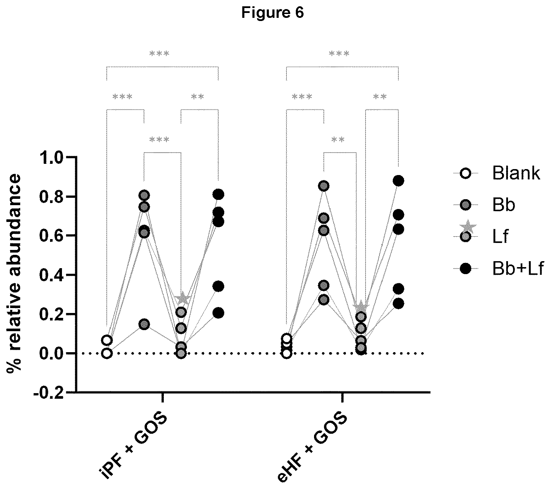

**Figure 6****:** Successful colonization in an ex vivo gut model. The relative abundance (in %) of B. breve DSM 32583 (short: Bb), of L. fermentum CECT 5716 (short: Lf), and of the combination of both strains (short: Bb + Lf) in each composition such as in an intact protein formula (iPF) and in an extensive hydrolyzed formula (eHF) both combined with GOS.

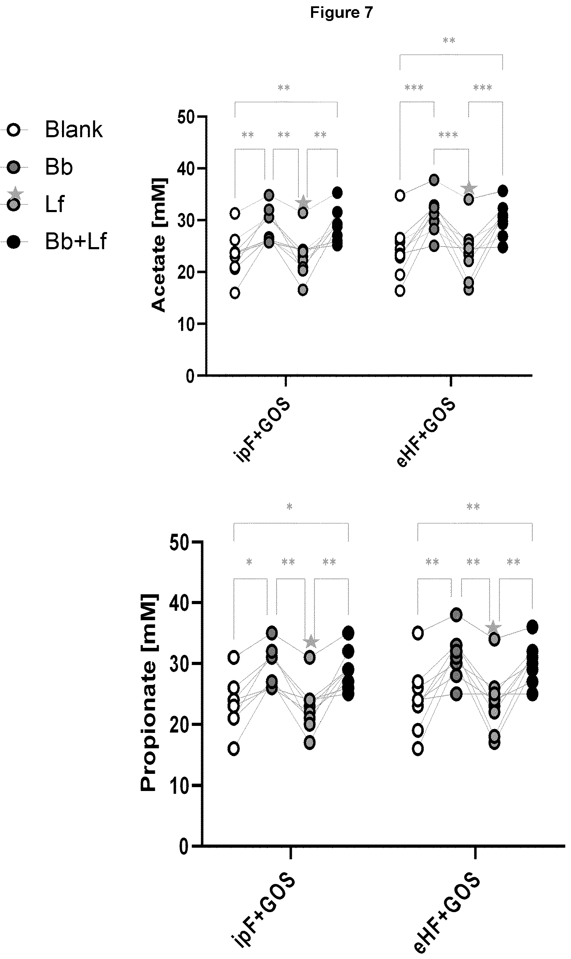

**Figure 7****:** SCFA profile of B. breve DSM 32583 (short: Bb) and/or L. fermentum in different infant formulas.

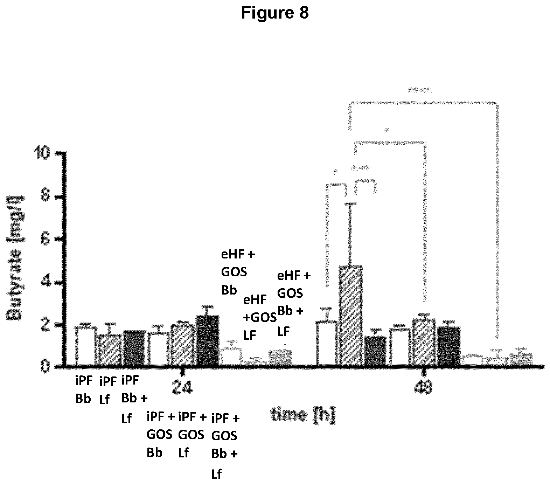

**Figure 8****:** Successful reduction of the butyrate production in intact protein and hydrolyzed formulae comprising the combination of Bb and Lf. Butyrate production (in mg/l) after 24 and 48 hours in an iPF formula, in an iPF formula combined with GOS and in an eHF formula also combined with GOS comprising B. *breve* DSM 32583 (short: Bb), *L. fermentum* CECT 5716 (short: Lf) and the combination of both strains (short: Bb + Lf). iPF = intact protein formula, eHF = extensive hydrolyzed formula; GOS = galacto-oligosaccharides. The labeling on the x-axis as can be depicted at "24 hours" is the same as at "48 hours".

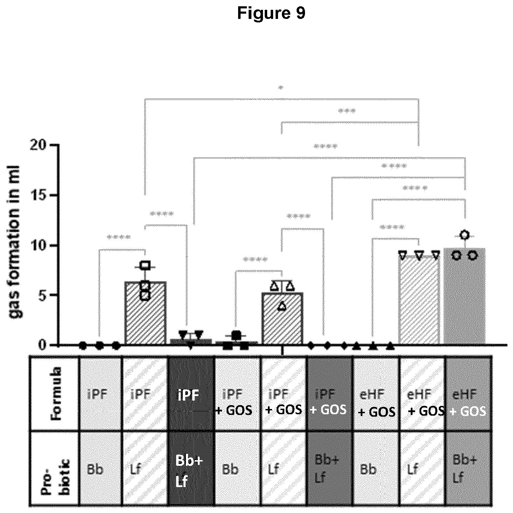

**Figure 9****:** Successful inhibition of the gas formation in intact protein formulae comprising the combination of Bb and Lf. Gas formation (in ml) in an iPF formula, an iPF formula combined with GOS and in an eHF formula also combined with GOS comprising B. *breve* DSM 32583 (short: Bb), *L. fermentum* CECT 5716 (short: Lf) and the combination of both strains (short: Bb + Lf). iPF = intact protein formula, eHF = extensive hydrolyzed formula; GOS = galacto-oligosaccharides.

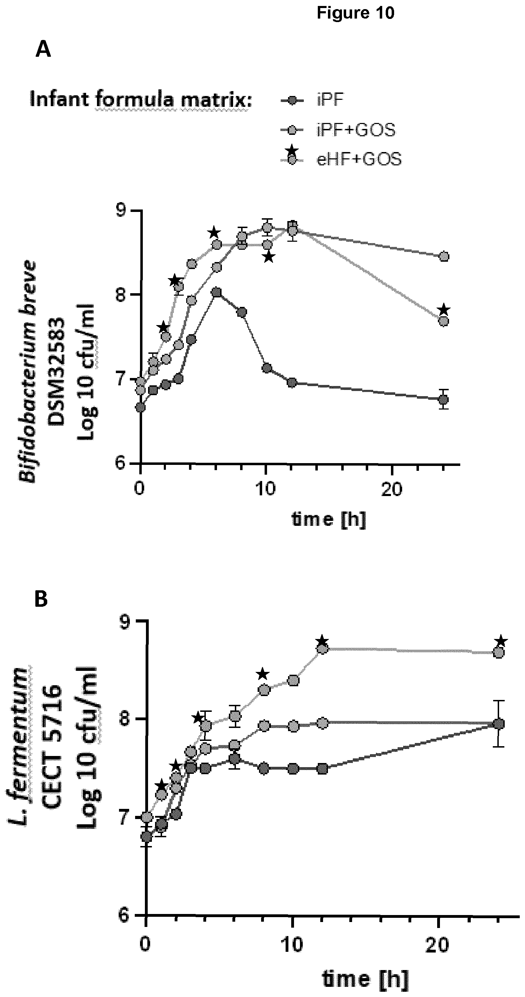

**Figure 10****:** Consistent growth rates of Bb and Lf. **(A)** Growth rate of *B*. *breve* DSM 32583 (in log10 cfu/ml) over time in an iPF formula, in an iPF formula combined with GOS and in an eHF formula also combined with GOS. **(B)** Growth rate of *L. fermentum* CECT 5716 (in log 10 cfu/ml) over time in an iPF formula, in an iPF formula combined with GOS and in an eHF formula also combined with GOS.

### DETAILED DESCRIPTION OF THE INVENTION

The inventors provide herein a novel *Bifidobacterium breve* strain, which was deposited with the DSMZ under the accession number DSM 32583.

Bifidobacteria are gram-positive, nonmotile, often branched, anaerobic bacteria. Bifidobacteria are one of the major genera of bacteria that make up the gastrointestinal tract microbiota in infants.

### Isolation from breast milk

The novel *B. breve* DSM 32583 has been isolated from human breast milk, which makes it particularly suitable and useful for the preparation of infant formulas / formulae (see **Example 2).**

### Successful colonization of the intestine of human infants in an ex vivo gut model

Early-life colonization of the intestinal tract is a dynamic process influenced by numerous factors. The impact of probiotic-supplemented infant formula on the composition and function of the infant gut microbiota is not well defined. It is however known that exogenous strains provided at early age are often unable to colonize the human intestine. To achieve a physiological effect in the intestine, it is necessary for strains to survive (pass through) the gastrointestinal passage and to settle in the intestine at least temporarily.

The novel B. breve DSM 32583 can colonize the intestine in said ex vivo gut model, as shown in **Figure 6****.** DSM 32583 survived the small intestinal passage and remained in the gut as demonstrated with said ex vivo gut model. The supplementation with probiotic Bb DSM 32583 increased the amount of Bifidobacteriaceae in most infants (see the appended Examples).

### Genome analysis

The genome of DSM 32583 has been sequenced to confirm its affiliation to Bifidobacterium (see **Example 1).**

### Pathogen defense

A further advantageous technical feature of the novel strain of the present invention, DSM 32583, is its advantageous anti-microbiological effect against different pathogens that are frequently involved in cases of infant infections. Said anti-microbiological effect is characterized by impairing or even inhibiting the growth of a pathogenic bacterium. Said surprising anti-microbiological effect (potential pathogen defense) has been examined by the present inventors by applying a well-established overlay assay as it is described in the appended Examples. The results of this assay are depicted in **Figure 1** (see also **Example 3)** which illustrates the remarkable anti-microbiological capabilities of the strain of the invention.

### Survival rate in infant formulas when having contact with oxygen

Anaerobic bacteria have sometimes difficulties to survive in the presence of oxygen (e.g. when preparing infant formulas). The particular strain of the present invention however has a survival rate after bottle preparation for an infant which makes it suitable for the preparation of infant formulas. In theory, the bacterial number added to the bottle before and after preparation should be the same. However, if there is a loss in the viability of the strain, the final concentration will be lower, since some of the anaerobic bacteria die after mixing in the aerobe setting.

The difference between the theoretical and the real concentrations at the end of the preparation is the survival of the strain (see **Figure 2** and **Example 4).** *B. breve* DSM 32583 has a survival rate of at least about 90% in the formula after bottle preparation, which qualifies said strain as a commercial strain for infant formulas.

### Production of riboflavin

*B. breve* DSM 32583 produces riboflavin, as described in **Example 5.**

### Production of CLNA and CLA

B. breve DSM 32583 also produces conjugated linolenic acid (CLNA) from linolenic acid (LNA) (see **Figure 4** and **Example 6).**

In addition, said *B*. *breve* DSM 32583 of the present invention is further capable of producing conjugated linoleic acid (CLA) from linoleic acid (LA) (see **Figure 3** and **Example 6).**

### Total lactate production

Said *B*. *breve* DSM 32583 is additionally capable of producing a total lactate content between about 400 to about 800 mg/L, between about 500 to about 700 mg/L, between about 550 to about 650 mg/L. Lactate was measured in the samples following the lactic acid UV-method kit instructions known to a person skilled in the art. In a preferred embodiment, said strain is capable of producing a total lactate content of about 596 mg/L (see **Table 2** and **Figure 5****,**

### Example 7).

As can be seen from **Figure 5** and **Table 2** the total lactate content in mg/L increases over time (measured from 8 to 48h) for each composition (see e.g. iPF and iPF+GOS for Bb in **Figure 5****)** in comparison to the same composition (without galacto-oligosaccharide (short: GOS)). When comparing the compositions to one another, the application of GOS into the compositions of the present invention, preferably into a nutritional composition as defined elsewhere herein, increases the lactate formation, thus *B*. *breve* DSM 32583 is capable of producing a total lactate content between about 600 to about 1000 mg/L, preferably a total lactate content of about 813 mg/L in comparison to the same composition, but not comprising GOS (see **Figure 5** and **Table 2).** Again, as can be seen from **Figure 5** and **Table 2 the** total lactate content in mg/L increases over time (measured from 8 to 48h) for each composition comprising GOS (see e.g. iPF+GOS and eHF+GOS for Bb in **Figure 5****).**

### Short chain fatty acid profile

The short-chain fatty acids (SCFAs) acetate, butyrate, and propionate (typically occurring in a 3:1:1 ratio) are quantitatively and metabolically the most important microbial end-products of the human colon fermentation process, as they display several physiological effects (Rivière A, Selak M, Lantin D, Leroy F and De Vuyst L (2016) Bifidobacteria and Butyrate-Producing Colon Bacteria: Importance and Strategies for Their Stimulation in the Human Gut. Front. Microbiol. 7:979). Changes in the bacterial ecosystem are associated with changes in the overall metabolic activity. Therefore, changes in metabolites upon probiotic supplementation formula either with intact or hydrolyzed protein was assessed. Supplementation with Bb DSM 32583 or the combination of Bb DSM 32583 +Lf (Lactobacillus fermentum CECT5716) yielded significantly higher levels of acetate, as well as propionate, compared to unsupplemented (blank) or Lf-supplemented conditions. SCFA profiles are optimized by B. breve DSM 32583, independent of food or other probiotics; said effect is likewise visible in the ex vivo system (see **Figure 7** and **Example 9).**

*B. breve* DSM 32583 of the invention may be comprised in any kind of culture medium for growing said strain therein. Preferably, said strain of the present invention is comprised in MRS culture medium. Said *B*. *breve* DSM 32583 may also be comprised in any kind of freezing medium, which may then refer to a frozen form of said strain. Further, said *B. breve* DSM 32583 as defined above may be comprised in any kind of lyophilization medium such as e.g skim milk or in any kind of freeze drying buffer for freeze drying said strain therein, which may refer to a freeze-dried form of said strain.

*B*. *breve* DSM 32583 of the present invention may also be comprised in a composition. Thus, the present invention also refers to a composition comprising said strain. In this context, the term "composition" refers to any kind of composition, which comprises said strain of the present invention, e.g. a nutritional composition, a cosmetic composition or the like. Said composition can be a liquid, a solid, a gel, a paste, an ointment, a capsule, a food product etc. Said cosmetic composition includes, but is not limited to, a toothpaste, a mouthwash, a cream (e.g. for the skin) an ointment, a balm, etc. preferably suitable for an infant (up to 36 months or 3 years as defined herein elsewhere) but it is also suitable for children with an age of 18, 17, 16, 15, 14, 13, 12, 11, 10, 9, 8, 7, 6, 5, or 4 years. Said composition may also be suitable for adults. The term "food product" comprises for example a food bar, a breakfast cereal, a snack food, a cake, a cookie, sweets, ice cream, a fruit composition (apple sauce, Quetschies, etc.) and in particular a formula, more preferably an infant formula, but also food product of the dairy industry, such as a yoghurt, a milk, acidified milk, a cheese, etc., preferably suitable for an infant (up to 36 months or 3 years as defined herein elsewhere) but it is also suitable for children with an age of 18, 17, 16, 15, 14, 13, 12, 11, 10, 9, 8, 7, 6, 5, or 4 years. It may also be suitable for adults. The composition comprising said strain may further comprise one or more other ingredients, such as a preservative, an ingestible support, a flavour, a nutritional component such as a (milk)protein, a carbohydrate, a lipid, a fat, GOS, FOS, vitamin etc., which is/are suitable for a food product, in particular for a food product intended and suitable for the ingestion by an infant (up to 36 months or 3 years as defined herein elsewhere), but it is also suitable for children with an age of 18, 17, 16, 15, 14, 13, 12, 11, 10, 9, 8, 7, 6, 5, or 4 years. It may also be suitable for adults. Preferred compositions of the present invention are defined elsewhere herein.

The composition of the present invention comprises *B. breve* DSM 32583 preferably in a viable form. The term 'viable' refers to a living microorganism which is capable of growing under suitable conditions and includes the microorganism in its resting period (e.g. in a non-reconstituted dry infant formula).

The composition of the invention may alternatively or additionally comprise the strain of the present invention in a "non-viable" form. In this context, the term "non-viable" refers to a microorganism, which is no longer capable of growing under suitable conditions and which is not capable of producing any metabolites. The term "non-viable" comprises partly or wholly disrupted cells, or otherwise inactivated *B*. *breve* DSM 32583 cells. The disruption can be done by known methods such as homogenization, sonication, or pressure cycling (like a French Press).

The term "partially disrupted cells" refers to cells of the present invention which are only partially broken up for example when mixing said cells with glass spheres (balls) as it is known to a person skilled in the art. These cells may still comprise intact cell membrane segments but also components of said cells are present. The term "wholly disrupted cells" refers to cells of the present invention, which are completely broken up for example by applying ultra-sonication or osmotic lysis.

The amount of viable cells can be expressed as bacteria counts, which refers to the number of viable bacteria contained per gram dry weight of the composition. The results may be given as bacteria counts/mL for liquids (e.g. when the composition of the present invention refers to a ready-to-drink preterm infant formula), and bacteria counts/g for solids (e.g. any other nutritional composition as described herein). The amount of viable cells is sometimes also expressed as colony forming units (CFU).

The composition of the invention may alternatively or additionally comprise a food matrix or a fluid fermented with said *B. breve* DSM 32583. The term food matrix or fluid fermented with said *B*. *breve* DSM 32583 comprises a substrate, wherein said substrate is fermented by said *B*. *breve* DSM 32583 as defined elsewhere herein. In this context, the term "substrate", which is comprised by the matrix or liquid / fluid to be fermented refers to any protein, fat and/or carbon source known to a person skilled in the art. "Fermented" means that the DSM 32583 of the invention has been grown with/on said substrate for a period of time which is sufficient for the bacteria to convert the substrate into metabolites, either partly or completely. The presence of metabolites may be determined by methods known in the art and may be reflected by a change of the pH in the medium, preferably by an acidification of the medium, e.g., from pH 7 to pH 6.5.

In terms of numerical quantities, said *B*. *breve* DSM 32583 of the present invention or said further bacterium as defined elsewhere herein may be present in said compositions as defined elsewhere herein in an amount corresponding to a value within a range between about 10³ bacterial counts or CFU per g dry weight of the composition to about 10¹¹ bacterial counts or CFU per g dry weight of the composition as defined elsewhere herein, such as about 10³, 10⁴, 10⁵, 10⁶, 10⁷, 10⁸, 10⁹, 10¹⁰ or even about 10¹¹. Said *B*. *breve* DSM 32583 of the present invention or said further probiotic bacterium as defined elsewhere herein may also be present in said compositions as defined elsewhere herein in an amount corresponding to a value within a range between about 10⁴ bacterial counts or CFU/g dry weight to about 10¹⁰ bacterial counts or CFU/g dry weight of the composition, between about 10⁵ bacterial counts or CFU/g dry weight to about 10⁹ bacterial counts or CFU/g dry weight of the composition, between about 10⁶ bacterial counts or CFU/g dry weight to about 10⁸ bacterial counts or CFU/g dry weight of the composition as defined elsewhere herein.

Said *B. breve* DSM 32583 as defined elsewhere herein, which may be comprised by said composition of the present invention and present in a viable form, may be preserved in order to maintain its viable cells, i.e. the reduction in the viability of said strain is decreased, at least in part. Methods of achieving this are well known. Preservation may include drying, preferably industrial scale drying, such as spray-drying or freeze-drying. Thus, said *B*. *breve* DSM 32583 as defined elsewhere herein, which may be comprised by said composition of the present invention may be spray-dried and/or freeze-dried, preferably freeze-dried. Also comprised herein, is the composition of the present invention comprising *B*. *breve* DSM 32583, wherein said composition contains a food matrix or fluid fermented with said *B*. *breve* DSM 32583 being spray-dried and/or freeze-dried. In this context, *B. breve* DSM 32583 cells may be in a viable form.

Said composition as defined elsewhere herein is preferably selected from a nutritional composition, a convenience food product, a nutritional supplement, or a care product. In a preferred embodiment, said compositions as defined herein are "Bio" or "organic" compositions, which may carry the organic seal being in existence in Germany since 2001, requirements hereto set out in the European Organic Regulation (EU) 2018/848 for example not using any genetic engineering and/or not being irradiated.

A "nutritional composition" means a substance or composition that satisfies at least a portion or all of a subject's nutrient requirements per day. Said nutritional composition is usually to be taken enterally, by a feeding tube directly into the stomach or orally. Preferably, a nutritional composition is for oral use. "Nutritional composition(s)" may refer to liquids, powders, gels, pastes, solids, concentrates, suspensions, or ready-to-use forms of enteral formulas, oral formulas, formulas for infants, for children and/or for adults. Such nutritional composition of the present invention is preferably selected from the group consisting of a preterm infant formula, a low birth weight infant formula, an infant formula, a follow-on infant formula, an enteral nutrition formula, a growing up milk, and an infant food. Preferably, said nutritional composition is in form of a powder or a liquid. Said nutritional composition according to the present invention may comprise intact milk and/or plant protein, or hydrolysed milk and/or plant protein.

The term "preterm infant formula" means an infant formula intended for a preterm infant. In this context, the term "preterm infant" means an infant born prior to 37 weeks gestational age, typically from about 26 to about 34 weeks gestation. The term "low birth weight (LBW) infant" means an infant having a liveborn weight less than 2,500 g at birth, including those less than 1.8 kg at birth. A preterm infant formula of the present invention may refer to a ready-to-drink liquid.

The term "infant feeding formula" or "infant formula", (short: IF) or the like means a foodstuff intended for particular nutritional use by infants during the first months of life, preferably the first four to six months of life and satisfying by itself the nutritional requirements of this category of person (European Commission 2016/127 of 25 September, 2015).

"Follow-on infant formula" (short: FOF) means according to the European Commission 2016/127 of 25 September, 2015, a foodstuff intended for particular nutritional use in combination with a diversified diet by infants aged over 6^{th} month onwards. Said foodstuff constitutes the principal liquid element in the progressively diversified diet of this category of person.

The infant formula and the follow-on infant formula can either be in the form of a liquid, ready-to-consumer (ready to use) or concentrated, or in the form of a dry powder that may be reconstituted to form a formula upon addition of water, preferably in the form of a dry powder. Such formulae are well-known in the art. The infant formulae for use herein, including the exemplified formulae described herein, can therefore be prepared by any of a variety of known or otherwise effective formulation or manufacturing methods.

The term "enteral nutrition formula" means a product which nourishes a subject. Said nutrition formula is to be taken enterally via the human gastrointestinal tract or digestive tract, and it usually includes a lipid or fat source and a protein source. Preferably, the enteral nutrition formula is a complete nutrition mix that fulfils all or most of the nutritional needs of a subject.

The term "growing-up milk" (in short: GUM) or "toddler milk" means a milk-based beverage adapted for the specific nutritional needs for infants more than one year old, usually up to 3 years. FOFs and GUMs can be fed to the young children only in combination with increasing amounts of other baby foods as defined elsewhere herein such as pureed fruits, vegetables and other foodstuffs as the process of weaning progresses.

The term "infant food" or "baby food" means a foodstuff intended for particular nutritional use by infants during the first months or years of life, preferably within the first 3 years or 36 months. Infant food in the context of the present invention includes, but is not limited to, a slurry, a porridge, pureed fruits, vegetables and other foodstuffs.

The terms "preterm infant formula", "infant formula", "follow-on infant formula", "enteral nutrition formula", "growing up milk", "infant food" and the like, are to be understood as "man-made", i.e. synthetic nutritional compositions, and do not encompass human milk. Infant formulae, follow-on infant formulae and growing-up milks as well as infant foods which may be aimed at different age groups are known. These infant formulae, follow-on infant formulae and growing up milks as well as infant foods aim to meet the requirements of infants and young children at the different ages.

Said convenience food product as another composition of the present invention is preferably selected from a cereal, a gummy, a cookie or a candy.

Said nutritional supplement as another composition of the present invention is preferably selected from an infant food supplement, a maternal food supplement, a suspension or a human milk fortifier. The nutritional supplements of the present invention may be fed to the infant and/or consumed by the mother of said infant. Nutritional supplements consumed by the mother of said infant may refer to a maternal food supplement as defined herein. Nutritional supplements fed to said infant may refer to an infant food supplement or a human milk fortifier as defined herein. A suspension as defined herein may be fed to said infant and/or consumed by said mother of said infant.

The nutritional supplement can be presented in any form (a liquid such as ready-to-use beverage, or a high energy gel or high energy snack bar served as a tablet, or a powder) and is consumed by said infant / mother of said infant next to consuming regular food. By consuming said maternal food supplement as defined herein the human milk of the mother becomes richer and more nutritious for said infant as defined herein which is then breast fed. In a preferred embodiment, said maternal food supplement is a powder, capsule or suspension.

A suspension, preferably an oil suspension or an aqueous suspension, may also be comprised under the term of nutritional supplement. An oil suspension - such as a vegetable oil suspension - comprising said B. breve DSM 32583, optionally comprising vitamin D or essential further components (e.g. lutein, DHA and/or ARA) or other probiotic bacterial strains may be preferred.

The term "human milk fortifier" means a supplement used to supplement the calories, protein, minerals and vitamins in human milk fed to preterm infants or infants with a low birth weight. Human milk fortification is generally used for all infants who require tube feeding of human milk and for a few infants who require fluid restriction. A human milk fortifier is preferably fed to preterm infants who require additional nutrients to support their growth. Human milk fortifier as defined herein is preferably in the form of a powder or a liquid, most preferably a powder.

Said care product as the final composition of the present invention is preferably selected from an oral hygiene care product or a skin care product. The oral hygiene care product preferably refers to a tooth paste. The skin care product preferably refers to a skin cream, body care oil or a moisturizing wipe (also called moisturizing tissue) or a diaper insert.

In a further preferred embodiment of the present invention, the composition as defined elsewhere herein comprises a further bacterium, preferably a bacterium which is also suitable to be fed to an infant, such as a probiotic bacterium. Thus, the composition of the invention may further comprise at least one probiotic bacterium from any one of the family of Bifidobacteriaceae, Carnobacteriaceae, Enterococcaceae, Lactobacillaceae, Streptococcaceae or Leuconostocaceae, preferably from the family of *Lactobacillaceae* or Bifidobacteriaceae, even more preferably from the family of *Lactobacillaceae.* The composition of the invention may further comprise at least one probiotic bacterium from any one of the genus *Carnobacterium, Enterococcus, Limosilactobacillus* and *Lacticaseibacillus* (previously *Lactobacillus*), *Lactococcus, Leuconostoc, Oenococcus, Pediococcus, Streptococcus, Tetragenococcus, Vagococcus* and *Weissella,* the genus Limosilactobacillus being preferred. More preferably, the at least one probiotic bacterium additionally comprised by the composition as defined herein is *Limosilactobacillus fermentum,* and/or *Lacticaseibacillus rhamnosus,* or any mixture thereof even more preferably, *Limosilactobacillus fermentum,* most preferably the further probiotic bacterial strain is *Limosilactobacillus fermentum* which is deposited with the CECT under the accession number CECT5716 (short. *L. fermentum* CECT 5716 or CECT 5716 strain or the like). In case a further Bifidobacterium species is additionally present in the defined composition of the present invention, it is preferably selected from the group consisting of *B*. *infants, B. lactis, B. bifidum, B. catenulatum, B. adolescentis, B. thermophilum, B. gallicum, B. animalis, B. angulatum, B. pseudocatenulatum, B. thermacidophilum* and *B. longum* or any mixture thereof.

The composition according to the invention may contain from 10³ to 10¹¹, such as about 10³, 10⁴, 10⁵, 10⁶, 10⁷, 10⁸, 10⁹, 10¹⁰ or even about 10¹¹ bacterial counts or CFU per g of composition on a dry weight basis of said further probiotic bacterial strain as defined elsewhere herein. Preferably, *L. fermentum* CECT 5716 as further probiotic bacterial strain as defined elsewhere herein may also be present in said compositions as defined above in an amount corresponding to a value within a range between about 10⁴ bacterial counts or CFU/g dry weight to about 10¹⁰ bacterial counts or CFU/g dry weight of the composition, between about 10⁵ bacterial counts or CFU/g dry weight to about 10⁹ bacterial counts or CFU/g dry weight of the composition, between about 10⁶ bacterial counts or CFU/g dry weight to about 10⁸ bacterial counts or CFU/g dry weight of the composition as defined elsewhere herein.

The abovementioned definitions as disclosed for *B*. *breve* DSM 32583 may also be applicable to said further probiotic bacterium, particularly to *L. fermentum* CECT 5716 which may also be comprised within the composition of the invention comprising said *B*. *breve* DSM 32583.

In a preferred embodiment, the composition as defined herein, such as a nutritional composition, a convenience food product, a nutritional supplement, or a care product, comprises any one of a non-digestible prebiotic oligosaccharide, a long-chain polyunsaturated fatty acid (short: LC-PUFAs), a vitamin, a mineral, a protein, a fat, or a combination thereof. In the context of the present invention, the term "prebiotic" preferably refers to one or more non-digestible oligosaccharides. Advantageously, the non-digestible oligosaccharide is water-soluble.

More specifically, the non-digestible prebiotic oligosaccharide being comprised by the present composition as defined herein may comprise any one of fructo-oligosaccharide, non-digestible dextrin, galacto-oligosaccharide, xylo-oligosaccharide, arabino-oligosaccharide, arabinogalactooligosaccharide, gluco-oligosaccharide, glucomannooligosaccharide, galactomanno-oligosaccharide, mannanoligosaccharide, chito-oligosaccharide, uronic acid oligosaccharide, sialyl-oligosaccharide, fuco-oligosaccharide, bovine milk oligosaccharide (BMOs), or human milk oligosaccharide (HMOs), which are structurally identical to the oligosaccharides in actual human milk and are produced during the whole lactation period. In a preferred embodiment the non-digestible prebiotic oligosaccharide comprises HMOs.

The present non-digestible oligosaccharide comprised in said compositions as defined herein is most preferably galacto-oligosaccharides (short: GOS), in particular β-galacto-oligosaccharides. In a particular preferred embodiment the present composition comprises between about 0.2 g/100 ml to about 0.8 g/100 ml GOS.

Additionally or alternatively to non-digestible prebiotic oligosaccharides, the composition may comprise long-chain polyunsaturated fatty acids. LC-PUFAs may comprise docosahexaenoic acid (short: DHA), a linoleic acid (short: omega-6), linolenic acid (short: omega-3), eicosapentaenoic acid (short: EPA), arachidonic acid (short: ARA), or any combination thereof. The LC-PUFA may be provided as free fatty acids, in triglyceride form, in diglyceride form, in monoglyceride form, in phospholipid form, or as a mixture of one of more of the above. Preferably, the composition comprises a long-chain polyunsaturated fatty acid which is any one of DHA, ARA, or a combination thereof. The amount of LC-PUFA in the composition may be at least about 5 mg/100 kcal. The composition may comprise LC-PUFA in an amount from about 5 mg/100 kcal to about 140 mg/100 kcal. Preferably, the composition may comprise LC-PUFA in an amount from about 10 mg/100 kcal to about 70 mg/100 kcal, most preferably between about 15 mg/100 kcal to about 47 mg/100 kcal.

The composition of the invention (preferably the nutritional composition) may comprise about 5 mg/100 kcal to about 100 mg/100 kcal of DHA. Preferably, the composition may comprise about 10 mg/100 kcal to about 50 mg/100 kcal of DHA. The composition may comprise about 5 mg/100 kcal to about 140 mg/100 kcal of ARA. Preferably, the composition may comprise about 10 mg/100 kcal to about 70 mg/100 kcal of ARA. The composition may comprise both DHA and ARA. The weight ratio of ARA:DHA may be between about 1.5:1. The composition may comprise oils containing DHA and/or ARA. The DHA and ARA may be in a natural form or in a refined form.

Additionally or alternatively, the composition of the present invention (the nutritional composition being preferred) may contain all vitamins and minerals understood to be essential in the daily diet and in nutritionally significant amounts. Minerals are usually added in salt form. The infant formula may optionally contain other substances which may have a beneficial effect such as folate, choline, nucleotides, nucleosides, and the like.

Additionally or alternatively, the proteins that may be utilized in the nutritional composition, in the convenience product or in the nutritional supplement or even in the care product include any proteins or nitrogen source suitable for human consumption. Such proteins are well known by those skilled in the art and can be readily selected when preparing such products. The composition according to the present invention may contain a protein source in an amount of between about 1.5 to about 3 g/100 kcal, preferably between about 1.6 to about 2.8 g/100 kcal. Also preferred as a protein additionally comprised by the compositions as defined herein is any one of a milk fat globule membrane protein (MFGMP), a hydrolysate, a collagen peptide or a cytokine. For the present invention a protein may also include peptides and free amino acids. The proteins may be intact or hydrolysed or a mixture of intact and hydrolysed proteins.

Additionally or alternatively, examples of suitable fat sources / fats typically incorporated into said compositions include high oleic safflower, sunflower oil, soy oil, fractionated coconut oil (medium chain triglycerides, MCT oil), corn oil, canola oil, coconut, palm and palm kernel oils, marine oil, cottonseed oil, bovine or goat milk fat and specific fatty acids as defined elsewhere herein such as DHA and ARA. The composition according to the present invention may contain a fat source in an amount of between about 3 to about 6 g/100 kcal, preferably between about 3.5 to about 5.5 g/100 kcal.

The carbohydrates that may also be used in said compositions can vary widely. Examples of carbohydrates suitable for (preterm) infants typically include hydrolyzed corn starch, maltodextrin, saccharose, glucose polymers, sucrose, corn syrup, corn syrup solids, rice syrup, glucose, fructose, lactose, high fructose corn syrup and non-digestible prebiotic oligosaccharides such as GOS as defined elsewhere herein. Any single carbohydrate listed above, or any combination thereof, as appropriate may be utilized. Preferably, the carbohydrate additionally comprised by the compositions defined herein besides GOS is lactose. The composition according to the present invention may contain a carbohydrate source in an amount of between about 5 to about 15 g/100 kcal, preferably between about 7 to about 11 g/100 kcal.

In a most preferred embodiment, the compositions, in particular a nutritional composition, as defined elsewhere herein comprises at least any one of:
- a total energy content of between about 450 to about 540 kcal/100 g, preferably between about 470 to about 520 kcal/100 g, most preferably between about 505 to about 515 kcal/100 g based on dry weight of the composition;
- between about 5 to about 22 wt% (w/w)% of a protein source, preferably between about 9 to 13 wt% of a protein source based on dry weight of the composition;
- between about 40 to about 66 wt% of a carbohydrate source, preferably between about 47 to 56 wt% of a carbohydrate source based on dry weight of the composition;
- between about 1 to about 12 wt% of a non-digestible oligosaccharide (in particular GOS), preferably between about 2 to 6 wt% of a non-digestible oligosaccharide (in particular GOS) based on dry weight of the composition;
- between about 20 to about 34 wt% of a fat source, preferably between about 24 to 28 wt% of a fat source based on dry weight of the composition;
- between about 0.057 to about 0.106 wt% of an ARA source, preferably between about 0.059 to 0.104 wt% of an ARA source based on dry weight of the composition; or
- between about 0.041 to about 0.164 wt% of a DHA source, preferably between about 0.059 to 0.114 wt% of a DHA source based on dry weight of the composition.

In an alternative most preferred embodiment, the compositions, in particular a nutritional composition, as defined elsewhere herein comprises at least any one of:
- a total energy content of between about 450 to about 540 kcal/100 g, preferably between about 470 to about 520 kcal/100 g, most preferably between about 505 to about 515 kcal/100 g based on dry weight of the composition;
- a protein source in an amount of between about 1.5 to about 3 g/100 kcal, preferably between about 1.6 to about 2.8 g/100 kcal;
- a carbohydrate source in an amount of between about 5 to about 15 g/100 kcal, preferably between about 7 to about 11 g/100 kcal;
- a fat source in an amount of between about 3 to about 6 g/100 kcal, preferably between about 3.5 to about 5.5 g/100 kcal;
- a DHA source in an amount of between about 5 mg/100 kcal to about 100 mg/100 kcal, preferably, between about 10 mg/100 kcal to about 50 mg/100 kcal of DHA; or
- an ARA source in an amount of between about 5 mg/100 kcal to about 140 mg/100 kcal, preferably between about 10 mg/100 kcal to about 70 mg/100 kcal.

In a particular embodiment, if the nutritional composition is a preterm infant formula, which is a ready-to-drink liquid, said preterm infant formula, as defined elsewhere herein comprises at least any one of:
- a total energy content of between about 70 to about 86 kcal/100 ml, preferably between about 76 to about 80 kcal/100 ml;
- between about 2 to about 6 g/100 kcal of a protein source, preferably between about 3.2 to 3.65 g/100 kcal of a protein source;
- between about 9 to about 12 g/100 kcal of a carbohydrate source, preferably about 10.5 g/100 kcal of a carbohydrate source;
- between about 4 to about 6 g/100 kcal of a fat source, preferably between about 4.85 to 5.1 g/100 kcal of a fat source;
- between about 30 to about 40 mg/100 kcal of an DHA source, preferably between about 33 to 36 mg/100 kcal of an DHA source; or
- between about 44 to about 63 mg/100 kcal of a ARA source, preferably between about 50 to 55 mg/100 kcal of a ARA source.

The composition according to the invention is preferably intended for an infant, optionally wherein said infant is a preterm infant. The term "infant" includes a child with an age of 3 years or 36 months or below. The term "infant" as used herein, includes a toddler at that age, actual or corrected, including individuals from 0 to 36 months of age, actual or corrected. The term "infant" comprises a "preterm infant" as defined herein or a "full-term infant". According to the present invention, the infant may be a preterm infant. In other words, said composition may be suitable for an infant (preterm or full-term infant) according to the present invention. Preferably, said infant to which the composition is to be fed to / is suitable for, is an infant (preterm and/or full-term infant) receiving human milk. Also preferably, said infant to which the composition is to fed to / is suitable for, is a preterm infant or a preterm infant receiving human milk. Said infant to which the composition preferably comprising *B*. *breve* DSM 32583 and *L. fermentum* CECT 5716 as defined elsewhere herein, is to be fed to / is suitable for is an infant, preferably receiving human milk, with an age below 6^{th} months, since in these first months of life, decreased levels of butyrate are particularly favorable in infants, preferably receiving human milk.

A preterm infant formula is intended for preterm infant being born prior to 37 weeks gestational age. An infant formula is intended for a full-term infant during the first months of life, preferably the first 4 to 6 months of life. A follow-on formula is intended for a full-term infant over the age of 6 months. A growing-up milk is intended for a full-term infant (toddler) with an age of 12 months or above, usually up to 3 years. If an infant food is fed, said infant refers to a full-term infant during the first years of life, particularly an infant (toddler) up to 36 months of age.

Said composition may particularly be suitable for enteral feeding to said infant. The composition is enterally fed, and the term "enteral" is intended to refer to the delivery directly into the gastrointestinal tract of the infant. Most preferably, said composition according to the present invention is fed to said infant orally.

In a further preferred embodiment, said infant as defined herein to which the composition is suitable for, is delivered by cesarean section. Alternatively, the infant may have been vaginally delivered. To limit the negative impact associated with the delivery by caesarean section on the development of an early bifidogenic intestinal microbiota, it is preferred that the infant formula of the present invention is fed at least within the first hours after delivery. Preferably, said infant as defined herein to which the composition comprising *B*. *breve* DSM 32583 and *L. fermentum* CECT 5716, is suitable for, is delivered by cesarean section. Particularly, to limit the negative impact associated with the delivery by caesarean section on the development of an early bifidogenic intestinal microbiota, such as an association with increased butyrate excretion (Mueller et al. 2021, BJOG 128(8):1293-1303), it is preferred that the composition suitable for an infant as defined herein, particularly for an infant with an age below 6^{th} months which receives human milk, is to be fed to said infant at least within the first hours after delivery, if said infant is delivered by cesarean section.

The composition may be in any form known in the art to be suitable for such feeding, such as in solid form, in semi-solid form or in liquid form. Preferably, the composition as defined elsewhere herein is in the form of a reconstitutable powder, a tablet, a flake, a softgel, a creme, a suspension or a ready-to-use liquid. A "reconstitutable powder" is a powder which is reconstituted with a suitable aqueous fluid, typically water or milk, to obtain / form a liquid composition for immediate oral or enteral use. Such powder includes spray dried, agglomerated, dry mixed or other known or otherwise effective particulate form. The quantity of such powder required to produce a volume suitable for one serving can vary. Preferably, the present composition, when in the form of a powder, a tablet, a flake, a creme, a softgel, a suspension or a ready-to-use liquid is contained within a container, preferably a stick or stickpack or a sachet. A "ready-to-use" liquid as used herein refers to a liquid suitable for feeding an infant right away, which is easy to drink or eat, and wherein no other components or additional water is/are added. A ready-to-use liquid may comprise a ready-to-feed liquid or ready-to-drink liquid (such as a preterm infant formula).

In an even more preferred embodiment, the nutritional composition of the present invention as defined elsewhere is suitable as the sole nutrition source for said infant, in particular for said preterm infant as defined elsewhere herein. Since the nutritional compositions according to the present invention can be used as a sole source of nutrition, it comprises a protein source, a lipid source, a carbohydrate source, vitamins, and minerals in amounts sufficient to maintain an infant's health (i.e., to prevent malnutrition), and optionally comprise folate, choline, nucleotides, nucleosides, GOS, HMOs and the like.

The present invention may also comprise in a further embodiment a composition comprising said B. breve DSM 32583 as defined herein and additionally comprising *L. fermentum* CECT 5716. The present invention may also comprise the use of B. breve DSM 32583 and of *L. fermentum* CECT 5716 as both defined herein for the manufacture of such composition as well as a method for the manufacture of an infant formula comprising the step of formulating B. breve DSM 32583 and *L. fermentum* CECT 5716 as both defined herein in a nutritional composition.

*Limosilactobacillus* is a thermophilic and heterofermentative genus of lactic acid bacteria created in 2020 by splitting from *Lactobacillus.* Lactic acid bacteria are gram-positive bacteria that are able to produce lactic acid as the main end product after carbohydrate fermentation. *L. fermentum* CECT 5716 is one of the most promising gut bacteria, which also provides antiinflammatory and immunomodulatory properties when fed to infants within compositions such as infant formulae (Rodriguez-Sojo et al. (2021), Nutrients 13, 1016). The particular *L. fermentum* CECT 5716 has been isolated from human milk of healthy mothers, which makes it also particularly useful for the preparation of infant formulae. Having this preferred combination of the two gut bacteria, namely of said B. breve DSM 32583 and said *L. fermentum* CECT 5716, within such composition such as an infant formula, it was also found by the inventors that such combination of these two strains reduces the butyrate production of such strains - when compared to the production level of the single strains (see **Figure 8** and **Example 10).** The short-chain fatty acid (SCFA) butyrate is quantitatively and metabolically one of the most important microbial end-products of the human colon fermentation process, as they display several physiological effects (Rivière A, et al. (2016), Front. Microbiol. 7:979). An overproduction of butyrate may however promote allergies (Wopereis et al. 2017, Atopic dermatitis and inflammatory skin disease, volume 141, issue 4, pp. 1334-1342). The reduction of the butyrate production as found for this particular embodiment is independent of at least any one of non-digestible prebiotic oligosaccharides, LC-PUFAs, vitamins, minerals, proteins, fats as defined herein which may optionally be comprised within said composition of the present invention. In detail, **Example 10** shows that this reduction occurs irrespective of the infant formula (in iPF and eHF).

Additionally, the present invention may also comprise in another further embodiment a composition comprising said B. breve DSM 32583 as defined herein and additionally comprising *L. fermentum* CECT 5716 as also defined herein, wherein the protein source of the composition is an intact protein source. Further, the present invention may also envisage the use of B. breve DSM 32583 and of *L. fermentum* CECT 5716 for the manufacture of such composition as well as a method for the manufacture of an infant formula comprising the step of formulating B. breve DSM 32583 and *L. fermentum* CECT 5716 in a nutritional composition, wherein the protein source of said composition is an intact protein source.

It was also found by the inventors that said *B. breve* DSM 32583 is capable of inhibiting the gas formation/production of *L. fermentum* CECT 5716 (see **Figure 9****).** *L. fermentum* CECT 5716 is a well-established and safe bacterium that has been successfully used in infant formulae for years. Said bacterium is however capable of producing gas within infant formulae, which could result in colic complaints (colics), e.g. when a composition, such as an infant formula, comprising such strain is fed to a particularly sensitive infant. It is known that bacteria, which are likewise suitable as "probiotic" bacteria (bacteria suitable for the use in infant formulae) can reduce or even inhibit such a gas formation - studies have shown, however, that this inhibition or reduction results from a suppression of the growth of the gas-formers. The inventors have now shown that the gas formation of *L. fermentum* CECT 5716 can be inhibited by the novel *B. breve* DSM 32583 when both are present in a composition, without inhibiting its growth significantly, as can be seen in **Figure 9** and **Example 11.** The inhibition of the gas formation of *L. fermentum* CECT 5716 by *B. breve* DSM 32583 as found for this particular embodiment is independent of at least any one of non-digestible prebiotic oligosaccharides, LC-PUFAs, vitamins, minerals, fats as defined herein which may optionally be comprised within said composition of the present invention.

By inhibiting the gas formation when combining these two strains, the growth of *L. fermentum* CECT 5716 and/or of *B*. *breve* DSM 32583 itself is however not inhibited (see **Figure 10****).** Thus, the growth rate of *L. fermentum* CECT 5716 maintains over several hours even though its gas production is inhibited. This was contrary to the findings of Savino et al. (2009) Acta Paediatr. 2009 Oct;98(10):1582-8 and Simone et al. (2014), Biomed Res Int. 2014;2014:301053 which demonstrated that in general the gas formation of gas-forming coliforms is inhibited by inhibiting the growth of the gas former. **Example 12** shows additionally that the reduction of gas formation however depends on the protein source used in the composition of this particular embodiment of the invention, i.e. an intact protein source is able to exert this wanted effect, while a hydrolyzed protein source does not. Thus, such inhibition of the gas formation of *L. fermentum* CECT 5716 by *B. breve* DSM 32583 (if combined together within the composition of this particular embodiment of the invention) is achievable only in a composition as defined herein, such as an infant formula, in particular an intact protein formula (short: iPF), wherein the protein source of the composition is an intact protein source (see **Figure 9****).** Such particular effect is thus composition dependent with regard to the protein source, whereas the wanted effect does not occur in an extensive hydrolyzed formula (short: eHF).

In this particular embodiment of the invention, the protein source of the composition is an intact protein source. In this context, the term "intact protein" refers to non-hydrolyzed protein(s), which is/are not fragmented into its/their components such as amino acids and small peptides by known methods such as heating the proteins with hydrochloric acid and/or enzymes. Such intact proteins may refer to any proteins or nitrogen source suitable for human consumption such as a milk fat globule membrane protein (MFGMP). The composition as defined above, wherein the protein source of said composition is an intact protein source may contain an intact protein source in an amount of between about 1.5 to about 3 g/100 kcal, preferably between about 1.6 to about 2.8 g/100 kcal. The present invention may also comprise said composition, wherein the protein source of said composition is an intact protein source, which does not comprise hydrolyzed protein(s) / a hydrolyzed protein source as it is the case in an eHF.

With regard to the abovementioned particular embodiment of the invention, the composition, in particular a nutritional composition, as defined elsewhere herein preferably comprises at least any one of:
- a total energy content of between about 450 to about 540 kcal/100 g, preferably between about 470 to about 520 kcal/100 g, most preferably between about 505 to about 515 kcal/100 g based on dry weight of the composition;
- between about 5 to about 22 wt% (w/w)% of an intact protein source, preferably between about 9 to 13 wt% of an intact protein source based on dry weight of the composition;
- between about 40 to about 66 wt% of a carbohydrate source, preferably between about 47 to 56 wt% of a carbohydrate source based on dry weight of the composition;
- between about 1 to about 12 wt% of a non-digestible oligosaccharide (in particular GOS), preferably between about 2 to 6 wt% of a non-digestible oligosaccharide (in particular GOS) based on dry weight of the composition;
- between about 20 to about 34 wt% of a fat source, preferably between about 24 to 28 wt% of a fat source based on dry weight of the composition;
- between about 0.057 to about 0.106 wt% of an ARA source, preferably between about 0.059 to 0.104 wt% of an ARA source based on dry weight of the composition; or
- between about 0.041 to about 0.164 wt% of a DHA source, preferably between about 0.059 to 0.114 wt% of a DHA source based on dry weight of the composition.

Again, with regard to the abovementioned particular embodiment of the invention, the composition, in particular a nutritional composition, as defined elsewhere herein alternatively comprises at least any one of:
- a total energy content of between about 450 to about 540 kcal/100 g, preferably between about 470 to about 520 kcal/100 g, most preferably between about 505 to about 515 kcal/100 g based on dry weight of the composition;
- an intact protein source in an amount of between about 1.5 to about 3 g/100 kcal, preferably between about 1.6 to about 2.8 g/100 kcal;
- a carbohydrate source in an amount of between about 5 to about 15 g/100 kcal, preferably between about 7 to about 11 g/100 kcal;
- a fat source in an amount of between about 3 to about 6 g/100 kcal, preferably between about 3.5 to about 5.5 g/100 kcal;
- a DHA source in an amount of between about 5 mg/100 kcal to about 100 mg/100 kcal, preferably, between about 10 mg/100 kcal to about 50 mg/100 kcal of DHA; or
- an ARA source in an amount of between about 5 mg/100 kcal to about 140 mg/100 kcal, preferably between about 10 mg/100 kcal to about 70 mg/100 kcal.

Again, with regard to the abovementioned particular embodiment of the invention, if the nutritional composition is a preterm infant formula, which is a ready-to-drink liquid, said preterm infant formula, as defined elsewhere herein preferably comprises at least any one of:
- a total energy content of between about 70 to about 86 kcal/100 ml, preferably between about 76 to about 80 kcal/100 ml;
- between about 2 to about 6 g/100 kcal of an intact protein source, preferably between about 3.2 to 3.65 g/100 kcal of an intact protein source;
- between about 9 to about 12 g/100 kcal of a carbohydrate source, preferably about 10.5 g/100 kcal of a carbohydrate source;
- between about 4 to about 6 g/100 kcal of a fat source, preferably between about 4.85 to 5.1 g/100 kcal of a fat source;
- between about 30 to about 40 mg/100 kcal of an DHA source, preferably between about 33 to 36 mg/100 kcal of an DHA source; or
- between about 44 to about 63 mg/100 kcal of an ARA source, preferably between about 50 to 55 mg/100 kcal of a ARA source.

The definitions disclosed herein concerning the DSM 32583 strain of the present invention and concerning the composition comprising said strain are applicable, where needed, to all other compositions as defined above. The definitions disclosed herein concerning all compositions as defined herein are also applicable, where needed, to the corresponding use of said strain(s) for the manufacture of each composition defined herein and to the corresponding method for the manufacture of an infant formula comprising the step of formulating said strain(s) in a corresponding nutritional composition.

It is noted that as used herein, the singular forms "a", "an", and "the", include plural references unless the context clearly indicates otherwise. Unless otherwise indicated, the term "at least" preceding a series of elements is to be understood to refer, if not particularly defined differently, to one or more such as two, three, four, five, or more. The term "and/or" wherever used herein includes the meaning of "and", "or" and "all or any other combination of the elements connected by said term".

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified. The term "including" means "including but not limited to". "Including" and "including but not limited to" are used interchangeably.

The term "about" means plus or minus 10%, preferably plus or minus 5%, more preferably plus or minus 2%, most preferably plus or minus 1%.

Throughout the description and claims of this specification, the singular encompasses the plural unless the context otherwise requires. In particular, where the indefinite article is used, the specification is to be understood as contemplating plurality as well as singularity, unless the context requires otherwise.

A better understanding of the present invention and of its advantages will be gained from the following examples, offered for illustrative purposes only. The examples are not intended to limit the scope of the present invention in any way.

### EXAMPLES OF THE INVENTION

### Material and Methods

### Example 1: Whole genome sequencing.

*B*. *breve* DSMZ 32583 was deposited as DSM 32583 in the German Collection of Microorganisms.

DNA was extracted using CTAB (Murray & Thompson, 1980) and fragmented (Covaris sonicator model E220, Covaris, UK). Libraries were prepared using the TruSeq DNA kit. Sequencing was conducted on a MiSeq using a PE300 v3 cartridge, generating 8,943,755 raw reads. Reads were checked using fastqc v0.72 and adapter were removed using Clip v1.0.3. The genome was assembled using Unicycler v0.4.6.0 with default settings and annotated with PROKKA v1.13.3 both implemented in Galaxy.

The genome of DSMZ 32583 comprises 2,292,381 bp in 11 contigs (*N₅₀* = 657,788 bp), with a G+C content of 58.74%, 1,951 coding sequences (CDS), 2 rRNAs and 54 tRNAs. It shared 98.2% average nucleotide identity with the genome of type strain B. *breve* DSM 20213^{T} (GCA_001025175.1), confirming its affiliation to *Bifidobacterium.* Genome completeness was estimated to be 100% at the taxonomic level of family using CheckM v1.0.18.. No bacteriocin or similar genes were detected through BAGEL4 and BACTIBASE.

### Example 2: Isolation from human milk and identification.

*Bifidobacterium breve* DSM 32583 was isolated in the frame of a study to evaluate the lactobacilli and bifidobacterial diversity of human milk. Women were enrolled in this study according with the following criteria: (a) healthy women without present or past underlying conditions (including mastitis); (b) normal full-term pregnancy; and (c) absence of infant and/or maternal perinatal problems (including mastitis). Milk samples were collected in sterile tubes by manual expression using sterile gloves. Previously, nipples and surrounding skin were cleaned with soap and sterile water, and soaked in chlorhexidine. The first drops (~1 ml) were discarded. The milk and skin samples were obtained at day 7 after delivery and kept at 4°C until delivery to the laboratory, which happened within the first three hours after collection. The study from which this strain was isolated was approved by the Ethical Committee on Clinical Research of Hospital Clinico (Madrid).

Peptone water dilutions of the milk samples were plated in triplicate onto de Man, Rogosa, and Sharpe (MRS, Oxoid, Basingstoke, UK) supplemented with L-cysteine (0.5 g/L) (MRS-Cys) and TOS agar plates, which were incubated anaerobically (85% nitrogen, 10% hydrogen, 5% carbon dioxide) in an anaerobic workstation (MINI-MACS, DW Scientific, Shipley, UK) at 37°C for 48 h. In this study, presence of bifidobacteria in human milk was investigated. Colonies were obtained from all the milk samples in MRS-Cys plates. The MRS-Cys and TOS counts ranged from 1.45 × 10² to 1.25 × 10⁴ CFU/ml. Such bacterial concentration values are similar to that reported from hygienically-obtained human milk. No growth was detected on VRBA plates, which confirmed the hygienic collection of the milk samples. A total of 104 colonies were selected from MRS-Cys or TOS plates for further characterization. Subsequently, they were inoculated in MRS broth tubes, which were incubated aerobically to exclude those with fastidious incubation requirements (<10⁸ CFU/ml after 24 h at 37°C). The isolates were examined by phase-contrast microscopy to determine cell morphology and Gram-staining reaction, and tested for oxidase and catalase activities. All the Gram-positive and catalase-negative isolates with a typical bifidobacterial shape were identified to the genus level (*Bifidobacterium*) by demonstration of fructose-6-phosphate phosphoketolase (F6PPK) activity in cellular extracts.

### Example 3: Antimicrobial activity.

An overlay method previously described (Magnusson & Schnürer, 2001; Martin et al. 2005b) was used to determine the ability of the strain to inhibit the growth of different bacteria. After the incubation, zones of inhibition around the strain streaks were examined and halo's diameters were measured. The following bacteria were employed as indicator organisms: *Enterococcus faecium* P21, *Enterococcus faecalis* TAB28, *Listeria monocytogenes* ScottA, *List. monocytogenes* Ohio, *Listeria innocua* RdC, *Staphylococcus aureus* CECT 5191, *Staphylococcus epidermidis* CECT 231, *Salmonella cholerasuis* CECT 4155, *Sal. Cholerasuis* CECT 409, *Sal. cholerasuis* CECT 443, *Salmonella enteritidis* 4396, *Escherichia coli* CECT 4076 (O157:H7), *E. coli* RJM1, *E. coli* RJM2, *Klebsiella pneumoniae* CECT 142, *Klebsiella oxytoca* CECT 860T and *Proteus vulgaris* CECT 484 (see **Figure 1****).** The plates overlaid with bacterial indicators were incubated at 37°C for 48 h, while those overlaid with yeasts cells or fungal spores were incubated at 30°C for up to 120 h. The plates were examined for zones of inhibition around the strain streaks. All experiments assaying inhibitory activity were performed in triplicate.

In parallel, the strain was grown in MRS broth at 37°C until early stationary phase (A620 ~1.0). Preparation of cell-free supernatants and analysis of their bacteriocinogenic activity were performed as described (Martin et al., 2005b). The microorganisms employed as indicators of bacteriocinogenic activity were the Gram-positive bacteria previously used for determination of the antimicrobial spectrum: *E. faecium* P21, *E. faecalis* TAB28, *P. acidilactici* 347, *L*. *monocytogenes* ScottA, *L. monocytogenes* Ohio, *L. seeligeri* RdC, *S. aureus* CECT 5191 and S. *epidermidis* CECT 231. *B. breve* DSM 32583 showed a clear inhibitory antimicrobial activity against all indicator organisms used in this study (see **Figure 1****).** Subsequently, this strain was screened for production of bacteriocins and/or reuterin but it did not produce these antimicrobial compounds.

### Example 4: Survival of L. fermentum and B. breve DSM 32583 in the presence of oxygen.

Said experiment comprises simulating formula milk preparation with shaking process, which is called "Bottle-Test" (see **Figure 2****).** The first step was to mix a simulate formula milk with each strain at the commercial concentration (10⁷ CFU/g). For this purpose, counts in the freeze-dried strains were performed, in triplicate, to assure the concentration of each one at the beginning. Then, based on the previous freeze-dried bacterial counts, a pre-formula was prepared and adjusted to a concentration of 10⁹ CFU/g, using a precision balance. Once the concentration was confirmed, in triplicate, by plate counts in MRScysBP, a final formula milk, for each single strain or for their combination, was prepared at 10⁷ CFU/g concentration. Temperatures and incubation conditions were 37°C in anaerobiosis for 48h; for combinations, 37°C in anaerobiosis for 48h in TOS medium to detect *B*. *breve* DSM 32583 and 37°C in aerobiosis for 48h in MRScys to detect *L. fermentum.* With the bacteria's supplemented formula, baby bottles were prepared by preparing the infant formula according to manufacturer instructions and shaking under aerobic conditions.

### Example 5: Production of riboflavin and folate.

After cultivation in MRS, *B*. *breve* DSM 32583 was washed 3 times with saline solution (0.85 % w/v NaCl), resuspended in this solution at the original culture volume and used to inoculate at 4 % v/v folate, riboflavin or vitamin B12-free culture medium that were then incubated without agitation at 37°C for 18 h. After growth, this washing-resuspension procedure was repeated and the resulting LAB solution was used to inoculate at 2% v/v fresh vitamin-free medium. This last step was repeated 7 times and after the last incubation, samples were taken to determine extra- and intra-cellular vitamin concentrations. A sample (500 µl) of LAB grown vitamin-free medium was mixed with equal parts of protecting buffer for folates (0.1 M phosphate buffer, pH 6.8 containing 1.5% w/v ascorbic acid to prevent vitamin oxidation and degradation) or of a 1% (v/v) acetic acid solution for riboflavin, followed by immediate centrifugation for 5 min at 5000 × g. The supernatant was collected (extracellular sample) and the pellet was resuspended in 500 µl of protecting buffer, boiled (100°C) for 5 min, centrifuged for 6 min at 10000 × *g* and the supernatant was collected (intracellular samples). The extracellular samples were also boiled and centrifuged and all supernatants were stored at -70°C until used for vitamin quantification. Folate concentrations were determined using a previously described microbiological assay using *L*. *rhamnosus* NCIMB 10463 as the indicator strain (Laiño et al, 2012). Briefly, samples or different concentrations of HPLC grade folic acid were placed with the indicator strain and incubated statically during 48 h at 37°C in 96 well sterile microplates containing the folate-free medium. The optical density (OD) was read at 580 nm using a microplate reader and the folate concentration of the samples was determined by comparing the OD with those obtained with the standard curve prepared using commercial folic acid. Riboflavin concentrations were determined in the same manner using *L. rhamnosus* ATCC 7469 as the indicator strain grown in the riboflavin-free medium.

### Example 6: CLA and CLNA production.

### Bacterial strains, growth media and conditions:

Eight bifidobacterial strains were used in the study. The bacterial strains were grown overnight at 37°C in MRS broth supplemented with 0.05% (w/v) L-cysteine-HCL (Sigma) and 0.2% (w/v) Tween-80 (MRS-Cys broth) under anaerobic conditions in an anaerobic station. Three percent (v/v) of these cultures were transferred to fresh MRS-Cys broth (10 ml) containing free LA (0.5 mg/ml) and/or free ALA (0.5 mg/ml) and incubated at 37°C for 24 h under anaerobic conditions. The strains that showed CLA production in MRS-Cys broth after an initial qualitative screening were also tested for CLA and/or CLNA production, as described above, in 10% reconstituted skim milk (Scharlau, Sentmenat, Barcelona, Spain) supplemented with 0.05% (w/v) L-cysteine and 0.8% (w/v) casamino acids (milk-based medium).

### Qualitative screening of CLA producers by UV spectroscopy method:

Lipid isolation from culture media was carried out using a chloroform/methanol (2:1, v/v) solution according to Folch method modified by Iverson et al. (2001). The lipid residues obtained were subjected to a N₂ flow and remained dissolved in chloroform at -20°C until spectrophotometric analysis. For this analysis, lipid extracts (200 µl) from each sample were placed on a quartz 96-wells plate and total CLA was quantified at a wavelength of 233 nm in a spectrophotometer according to Rodriguez-Alcalá et al. (2011).

### Quantitative analysis of CLA and CLNA production:

The concentrations of CLA and CLNA in the culture media were determined using a direct methylation method. Heptadecanoic acid (C17:0) was added to the samples as an internal standard. The Fatty Acid Methyl Esters (FAMEs) were dissolved in n-hexane and determined by gas chromatography (GC) in a chromatograph equipped with a VF-23 column (30 m × 0,25 nm × 0,25 µm). For GLC analysis, the initial temperature was 80°C. Then, the temperature was increased to 170°C at 30°C/min, held at 170°C for 3 min, increased to 230°C at 30°C/min and, and finally held at 230°C for 7 min. Helius was used as the carrier gas at a pressure of 15 psig and with a split ratio of 1:50. The injection volume was 0.5 µl and the analysis time was 15 min. Peaks were identified by comparing the retention times of CLA methylated standards and by gas chromatography-mass spectrometry (GC/MS). CLA and CLNA concentrations were expressed as µg/ml and their conversion rates from LA and LNA were calculated using the formula [CLA/(CLA+LA)]×100 and [CLNA/(CLNA+LNA)]×100, respectively.

### Example 7: Total lactate production.

The Lactate production of the selected combinations (*L. fermentum, B. breve* DSM 32583, both) was measured with the same strain preparations. Measurement took place at the following time points: 8, 24, 48 h.

Lactate was measured in the samples following the D-lactic acid/L-Lactic acid UV-method kit instructions (r-biopharm/Roche).

Briefly, 1 mL aliquot of each tube was centrifuged for 10 min at 4°C and 16000 x g to separate the phases. After centrifugation, supernatants were diluted with water/puffer/something else based on the initial test performed to adjust the dilution. Diluted samples were analyzed following manufacturer instructions and samples that did not fulfill quality standard (difference in sample absorbance lower than 0.100 at 340 nm) were repeated. After the analysis, lactate values were corrected based on this dilution factor.

### Total lactate results were expressed in mg/L

**Table 2: Total lactate production for each composition comprising B. breve DSM 32583, L. fermentum CECT 5716 or the combination of both of them (1:1).**

| | **Total Lactate (mg/L)** | | |
|---|---|---|---|
| | 8h | 24h | 48h |
| Pre Bio (1:1) | 433.37 | 1394.45 | 2369.79 |
| Pre Bio DSM 32583 | 596.09 | 1514.03 | 1483.35 |
| Pre Bio *L. fermentum* | 221.08 | 349.26 | 493.64 |
| Pre Bio + GOS (1:1) | 825.62 | 2839.90 | 2233.68 |
| Pre Bio + GOS DSM 32583 | 813.31 | 1479.21 | 2146.31 |
| Pre Bio + GOS *L. fermentum* | 594.81 | 814.33 | 723.92 |

### Example 8: Ex-vivo gut model with infant fecal microbiome.

Infant formula with intact or hydrolyzed protein were subjected to a full passage through the oral, gastric and small intestinal phase, the latter involving absorption as described previously (Calatayud, Van den Abbeele et al. 2021). Quantity of protein and other nutrients in both formulas was comparable. Fecal material was collected with parental consent and ethical approval of the University Hospital Ghent (reference number B670201836585) from nine infants (2-8 weeks of age; three born by vaginal delivery and two born by caesarian section; exclusively breastfed). Fecal suspensions were prepared in phosphate buffer, to which reducing agents were added (K2HPO4 8.8 g/L; KH2PO4 6.8 g/L; sodium thioglycolate 0.1 g/L; sodium dithionite 0.015 g/L). The obtained suspensions were mixed with a cryoprotectant (modified version of (Hoefman, Pommerening-Röser et al. 2013) in a 1:1 (v:v) ratio, so that 7.5% fecal suspensions were obtained). The fecal suspensions were flash frozen and then preserved at -80°C (cryostock). Just before the experiment, fecal samples were defrosted and immediately added to the reactors. At the start of the short-term colonic incubation, the test ingredients (probiotic strains) were added to sugar-depleted buffered nutritional medium containing basal nutrients present in the colon. Finally, the cryopreserved fecal suspensions of each of the five donors were added (10% (v:v)). Control conditions had no probiotic addition. Reactors were incubated for 48h at 37°C, under continuous shaking (90 rpm) and anaerobic conditions. The incubations were performed in fully independent reactors with sufficiently high volume (70 mL) in order to not only ensure robust microbial fermentation, but also to allow the collection of multiple samples over time.

### Example 9: SCFA profiles.

The short-chain fatty acids (SCFAs) acetate, butyrate, and propionate (typically occurring in a 3:1:1 ratio) are quantitatively and metabolically the most important microbial end-products of the human colon fermentation process, as they display several physiological effects (Rivière A, Selak M, Lantin D, Leroy F and De Vuyst L (2016) Bifidobacteria and Butyrate-Producing Colon Bacteria: Importance and Strategies for Their Stimulation in the Human Gut. Front. Microbiol. 7:979). Changes in the bacterial ecosystem are associated with changes in the overall metabolic activity. Therefore, changes in metabolites upon probiotic supplementation formula either with intact or hydrolyzed protein was assessed. Supplementation with Bb DSM 32583 or the combination of Bb DSM 32583 +Lf (Lactobacillus fermentum CECT 5716) yielded significantly higher levels of acetate, as well as propionate, compared to unsupplemented (blank) or Lf-supplemented conditions. For acetate and propionate production, all infants showed higher levels upon Bb DSM 32583 supplementation. Overall, Bb and Bb+Lf supplementation showed a trend towards increasing SCFA levels, independent of formula matrix, compared to respective unsupplemented controls (p>0,05). Production of different acids lowered the pH, compared to unsupplemented conditions.

SCFA profiles are optimized by B. breve DSM 32583, independent of food or other probiotics; said effect is likewise visible in the ex vivo system (see **Figure 7****).**

### Example 10: Reduction of the butyrate production.

The short-chain fatty acid (SCFA) butyrate is quantitatively and metabolically one of the most important microbial end-products of the human colon fermentation process, as they display several physiological effects (Rivière A, et al. (2016), Front. Microbiol. 7:979). Changes in the bacterial ecosystem are associated with changes in the overall metabolic activity. Therefore, changes in metabolites upon probiotic supplementation formula either with intact or hydrolyzed protein was assessed. As can be seen from **Figure 8** supplementation with *L. fermentum* CECT 5716 (Lf) and *B*. *breve* DSM 32583 (Bb) yielded significantly lower levels of butyrate after 48 hours in the intact protein formula ("iPF / Bb + Lf"), in the iPF combined with GOS ("iPF + GOS / Bb + Lf") and in the eHF combined with GOS ("eHF + GOS / Bb + Lf") in comparison to the corresponding formulae only comprising one of these strains. Overall, Bb and Lf supplementation showed a trend towards decreasing butyrate levels, independent of formula matrix or other probiotics, compared to respective formulae only comprising Bb or Lf (p>0,05).

### Example 11: Inhibition of the gas formation.

*L. fermentum,* in particular *L. fermentum* CECT 5716 is capable of producing gas and is thus considered a gas producing coliform bacterium. The gas formation produced by such strain can lead to colic complaints (colics), e.g. when a composition, such as an infant formula, comprising such strain is fed to a human, preferably to a particularly sensitive infant. Therefore, changes in the gas formation upon probiotic supplementation formula either with intact or hydrolyzed protein was assessed. As can be seen from **Figure 9** the gas formation of *L. fermentum* CECT 5716 (Lf) in the intact protein formula (iPF) (see the third column "iPF / Lf" starting from the left) was significantly inhibited when using within the composition in addition to Lf, *B. breve* DSM 32583 (Bb) (see the fourth column "iPF / Bb + Lf" starting from the left). The same applies when using an iPF comprising GOS as prebiotic (see the sixth "iPF + GOS / Lf" and the seventh column "iPF + GOS / Bb + Lf" starting from the left). Overall, Bb + Lf supplementation showed a trend towards inhibited gas levels, dependent on the formula matrix, since such effect was however not achieved when using an extensive hydrolyzed formula (eHF), which contains instead of intact proteins hydrolyzed proteins. No gas formation was depicted in each formula only comprising *B*. *breve* DSM 32583 (see "iPF / Bb"; "iPF + GOS / Bb" and "eHF / Bb").

### Example 12: Growth rate.

Further, it was assessed whether the growth of such gas producer strain *L. fermentum* CECT 5716 (Lf) is decreased as known from other gas-forming coliforms (Savino et al. (2009) Acta Paediatr. 2009 Oct;98(10):1582-8 and Simone et al. (2014), Biomed Res Int. 2014;2014:301053). Eventhough the gas formation of *L. fermentum* CECT 5716 was inhibited, the growth rates (in cfu/ml) over time (in hours) of *B*. *breve* DSM 32583 (Bb) and Lf maintained for a certain period of time after an increase within the first about 10 hours (see **Figure 10A****:** iPF and eHF + GOS; **Figure 10B****:** iPF, iPF and eHF + GOS). The application of GOS was favorable for the growth rates of both strains over time.

## Claims

1. *Bifidobacterium breve* strain, which is deposited with the DSMZ under the accession number DSM 32583.

2. A composition comprising the *Bifidobacterium breve* strain of claim 1.

3. The composition of claim 2, wherein said *Bifidobacterium breve* strain is spray-dried or freeze-dried.

4. The composition of claim 2 or 3, wherein said *Bifidobacterium breve* strain is present in an amount between about 10³ to about 10¹¹ CFU per gram dry weight of the composition.

5. The composition of any one of claims 2 to 4, wherein said composition is selected from the group consisting of a preterm infant formula, an infant formula, a follow-on infant formula, an enteral nutrition formula, a growing up milk, and an infant food.

6. The composition of any one of claims 2 to 5, comprising a further bacterium.

7. The composition of claim 6, wherein said further bacterium is from the family of Lactobacillaceae or Bifidobacteriaceae.

8. The composition of any one of claims 2 to 7, further comprising any one of a non-digestible prebiotic oligosaccharide, a long-chain polyunsaturated fatty acid, (LC-PUFA), a vitamin, a mineral, a protein, a fat, or a combination thereof.

9. The composition of any one of claims 2 to 8, comprising at least any one of:
- a total energy content of between about 450 to about 540 kcal/100g based on dry weight of the composition;
- between about 5 to about 22 wt% (w/w)% of a protein source based on dry weight of the composition;
- between about 40 to about 66 wt% of a carbohydrate source based on dry weight of the composition;
- between about 1 to about 12 wt% of a non-digestible oligosaccharide based on dry weight of the composition;
- between about 20 to about 34 wt% of a fat source based on dry weight of the composition;
- between about 0.057 to about 0.106 wt% of an ARA source based on dry weight of the composition; or
- between about 0.041 to about 0.164 wt% of a DHA source based on dry weight of the composition.

10. The composition of any one of claims 2 to 9, wherein said composition is suitable as the sole nutrition source for an infant, e.g. a preterm infant.

11. Use of the *Bifidobacterium breve* strain DSM 32583 for the manufacture of a composition of any one of claims 2 to 10.

12. A method for the manufacture of an infant formula comprising the step of formulating the Bifidobacterium breve strain DSM 32583 in a nutritional composition.

## Patentansprüche

1. *Bifidobacterium breve*-Stamm, der bei der DSMZ unter der Zugangsnummer DSM 32583 hinterlegt ist.

2. Zusammensetzung, umfassend den *Bifidobacterium breve*-Stamm nach Anspruch 1.

3. Zusammensetzung nach Anspruch 2, wobei der *Bifidobacterium breve*-Stamm sprühgetrocknet oder gefriergetrocknet ist.

4. Zusammensetzung nach Anspruch 2 oder 3, wobei der *Bifidobacterium breve-*Stamm in einer Menge von etwa 10³ bis etwa 10¹¹ CFU pro Gramm Trockengewicht der Zusammensetzung vorliegt.

5. Zusammensetzung nach einem der Ansprüche 2 bis 4, wobei die Zusammensetzung ausgewählt ist aus der Gruppe bestehend aus einer Frühgeborenen-Säuglingsnahrung, einer Säuglingsnahrung, einer Folgenahrung, einer enteralen Ernährungsnahrung, einer Kleinkindermilch und einer Säuglingsnahrung.

6. Zusammensetzung nach einem der Ansprüche 2 bis 5, umfassend ein weiteres Bakterium.

7. Zusammensetzung nach Anspruch 6, wobei das weitere Bakterium aus der Familie der Lactobacillaceae oder der Bifidobacteriaceae stammt.

8. Zusammensetzung nach einem der Ansprüche 2 bis 7, ferner umfassend eines der folgenden: ein nicht verdauliches präbiotisches Oligosaccharid, eine langkettige mehrfach ungesättigte Fettsäure (LC-PUFA), ein Vitamin, ein Mineral, ein Protein, ein Fett oder eine Kombination davon.

9. Zusammensetzung nach einem der Ansprüche 2 bis 8, umfassend mindestens eines der folgenden:
- einen Gesamtenergiegehalt zwischen etwa 450 bis etwa 540 kcal/100 g, bezogen auf das Trockengewicht der Zusammensetzung;
- zwischen etwa 5 bis etwa 22 Gew.-% (w/w)% einer Proteinquelle, bezogen auf das Trockengewicht der Zusammensetzung;
- zwischen etwa 40 bis etwa 66 Gew.-% einer Kohlenhydratquelle, bezogen auf das Trockengewicht der Zusammensetzung;
- zwischen etwa 1 bis etwa 12 Gew.-% eines nicht verdaulichen Oligosaccharids, bezogen auf das Trockengewicht der Zusammensetzung;
- zwischen etwa 20 bis etwa 34 Gew.-% einer Fettquelle, bezogen auf das Trockengewicht der Zusammensetzung;
- zwischen etwa 0,057 bis etwa 0,106 Gew.-% einer ARA-Quelle, bezogen auf das Trockengewicht der Zusammensetzung; oder
- zwischen etwa 0,041 bis etwa 0,164 Gew.-% einer DHA-Quelle, bezogen auf das Trockengewicht der Zusammensetzung.

10. Zusammensetzung nach einem der Ansprüche 2 bis 9, wobei die Zusammensetzung als alleinige Nahrungsquelle für einen Säugling, z.B. einen Frühgeborenen, geeignet ist.

11. Verwendung des *Bifidobacterium breve*-Stamms DSM 32583 zur Herstellung einer Zusammensetzung nach einem der Ansprüche 2 bis 10.

12. Verfahren zur Herstellung einer Säuglingsnahrung, umfassend den Schritt des Formulierens des Bifidobacterium breve-Stamms DSM 32583 in einer Nahrungszusammensetzung.

## Revendications

1. Souche de *Bifidobacterium breve* déposée auprès de la DSMZ sous le numéro d'accession DSM 32583.

2. Composition comprenant la souche de *Bifidobacterium breve* selon la revendication 1.

3. Composition selon la revendication 2, dans laquelle la souche de *Bifidobacterium breve* est séchée par pulvérisation ou lyophilisée.

4. Composition selon la revendication 2 ou 3, dans laquelle la souche de *Bifidobacterium breve* est présente en une quantité d'environ 10³ à environ 10¹¹ UFC par gramme de poids sec de la composition.

5. Composition selon l'une quelconque des revendications 2 à 4, dans laquelle la composition est choisie dans le groupe constitué par une préparation pour nourrissons prématurés, une préparation pour nourrissons, une préparation de suite, une préparation nutritionnelle entérale, un lait de croissance et une préparation pour nourrissons.

6. Composition selon l'une quelconque des revendications 2 à 5, comprenant une bactérie supplémentaire.

7. Composition selon la revendication 6, dans laquelle la bactérie supplémentaire appartient à la famille des Lactobacillaceae ou des Bifidobacteriaceae.

8. Composition selon l'une quelconque des revendications 2 à 7, comprenant en outre un oligosaccharide prébiotique non digestible, un acide gras polyinsaturé à longue chaîne (LC-PUFA), une vitamine, un minéral, une protéine, une matière grasse ou une combinaison de ceux-ci.

9. Composition selon l'une quelconque des revendications 2 à 8, comprenant au moins l'un des éléments suivants:
- une teneur énergétique totale comprise entre environ 450 et environ 540 kcal/100 g, sur la base du poids sec de la composition;
- entre environ 5 et environ 22 % (w/w)% en poids d'une source de protéines, sur la base du poids sec de la composition;
- entre environ 40 et environ 66 % en poids d'une source de glucides, sur la base du poids sec de la composition;
- entre environ 1 et environ 12 % en poids d'un oligosaccharide non digestible, sur la base du poids sec de la composition;
- entre environ 20 et environ 34 % en poids d'une source de matières grasses, sur la base du poids sec de la composition;
- entre environ 0,057 et environ 0,106 % en poids d'une source d'ARA, sur la base du poids sec de la composition; ou
- entre environ 0,041 et environ 0,164 % en poids d'une source de DHA, sur la base du poids sec de la composition.

10. Composition selon l'une quelconque des revendications 2 à 9, dans laquelle la composition convient comme seule source nutritionnelle pour un nourrisson, par exemple un nourrisson prématuré.

11. Utilisation de la souche de *Bifidobacterium breve* DSM 32583 pour la fabrication d'une composition selon l'une quelconque des revendications 2 à 10.

12. Procédé de fabrication d'une préparation pour nourrissons, comprenant l'étape de formulation de la souche de *Bifidobacterium breve* DSM 32583 dans une composition nutritionnelle.
